# EUROPEAN PATENT SPECIFICATION

(11) **EP 0 788 874 B1**
(45) Date of publication and mention of the grant of the patent: **15.09.1999**
(21) Application number: 97300861.8
(22) Date of filing: 11.02.1997
(51) Int. Cl.: B32B 5/24, D04H 13/00

(54) **A laminated composite material, a method of making and products derived therefrom**
Schichtverbundmaterial, Verfahren zur Herstellung und davon abgeleitete Produkten
Matériau composite stratifié, procédé pour la production et des produits dérivés

(30) Priority: 12.02.1996 US 599909
(43) Date of publication of application: 13.08.1997
(73) Proprietor: McNEIL-PPC, Inc., Skillman, New Jersey 08558 (US)
(72) Inventor: Ulman, John, Woodbridge, NJ 07095 (US); Salerno, Catherine E., Millington, NJ 07946 (US); Costa, Rogerio, 126000-000-SP (BR)
(74) Representative: Mercer, Christopher Paul

(56) References cited:
- EP-A- 0 432 755
- DE-A- 4 243 012
- GB-A- 2 284 553
- US-A- 3 615 976
- US-A- 4 801 482
- US-A- 5 336 545

## Description

### FIELD OF THE INVENTION

The present invention relates to a novel, layered composite material, and more particularly to a gathered, ruffled or puckered, layered composite material. The composite material comprises a first layer of a flexible, reversibly elongatable material, such as for example a fabric, film, foam or combination thereof, bonded at spaced apart positions to at least one additional layer of a flexible material, such as for example a fabric, film, foam or combination thereof; wherein both layers are gathered, the gathers of one layer being aparallel to the gathers of the other layer. The layered composites of this invention may be used in the manufacture of various products including, but not limited to, sanitary absorbent products such as diapers, sanitary napkins, incontinence products, as well as bandages, wound dressings, surgical dressings, cushioning and bandages for the treatment of stasis ulcers, surgical drapes, underpads and the like.

### BACKGROUND OF THE INVENTION

The manufacture of flexible, gathered materials, by covering a tensioned elastic material with a second material, and securing the second material to the tensioned elastic material, followed by the step of releasing the tension or the elastic material, is known in the art. An early description of such a material, an intermittently bonded untensioned stretch laminate web, is given in US Patent 2,075,189 issued to Galligan et al, where two superposed continuous plies of rubber, one of which is under tension and longitudinally stretched, are passed between a pair of pressure rolls traveling at the same peripheral speed. One of the rolls is provided with relatively small or narrow projections in a desired pattern, the projections cooperating with the second roll to press together, into adhesive contact, small portions of the two plies of rubber; so that relatively closely spaced small areas of the superposed plies will be united in a pattern similar to that of the projections on the pressure roll. Harwood in US Patent 3,025,199, describes an untensioned stretch laminate web, specifically suggested for use in toweling, wiping material and expendable garment material. Harwood describes the formation of a scrim comprised of intersecting sets of threads or filaments that are bonded to each other at their points of intersection to form a reticulated reinforcing network, the threads or filaments being preferably resilient. A pair of nonwoven layers are attached preferably to opposite sides of the reinforcing network scrim. The laminate of nonwovens and scrim is subsequent subjected to a stretching operation in one or more directions to permanently expand the nonwoven layers. When the tensile forces of the stretching operation are removed, the reinforcing network restores itself and the outermost nonwoven plies exhibit Z-direction bulking in the areas where the nonwovens are not bonded to the reinforcing network.

Sisson in US Patents 4,107,364 and 4,209,563 describes a preferred embodiment, which is an untensioned stretch laminate comprised of at least one ply made substantially of relatively elastomeric synthetic polymeric filaments and at least one ply made of elongatable but relatively nonelastic synthetic polymeric filaments.

Ness in US Patent 4,525,407 discloses disposable diapers and surgical gowns incorporating untensioned stretch laminate composites comprised of an untensioned elastic member intermittently bonded to one or more unstretched less extensible substrates, the resulting laminate being rendered elastic by stretching. On stretching the laminate, the substrate undergoes permanent elongation and may become delaminated from any other substrate, but remains intermittently bonded to the elastic member. Upon release of the applied tensile forces, the elastic member causes puckering, i.e., Z-direction bulking, of the permanently elongated substrate between the bonding points.

US Patent 5,336,545 issued to Morman describes a composite material made by joining a nonelastic material which has been tensioned to neck it down to a narrower width and joined to an elastic sheet. The composite material is elastic in a direction parallel to the direction of neckdown and may be stretched in that direction to the breaking point of the necked material. Neither of the two materials comprising the composite become gathered.

Ruffled or gathered fabrics have also been disclosed, for example, in US Patent 4,720,415 issued to Taylor et al and by Buell et al in US Patent 5,151,092 wherein a traditional elastic member is applied to a backsheet that is then prestrained by mechanical stretching to permanently elongate the backsheet and stretch the elastic member. The elastic member on being released yields a ruffled or gathered material. Materials such as these include an elastomeric material which forms a stretchable elastic layer. To at least one side of this material while in a stretched condition there is attached another gatherable layer. Once the two layers have been attached to one another, the elastic layer is allowed to retract thereby gathering up and puckering the nonelastic gatherable layer to form a three dimensional material. US 4,891,258 to Fahrenkrug describes a stretchable composite structure wherein the stretchable layer may comprise a pervious elastomeric film.

The prior art of composites that are stretchable or contain stretchable components, requires the presence of a reversibly elongatable component, that provides reversible restoring forces, to be bonded to a relatively inelastic less easily extendible component, thereby creating a gathering, ruffling or puckering of the inelastic component; the gathering of the inelastic component being orthogonal to the direction in which the laminate or the elastic component is extended, i.e., in a direction cross to the direction of stretch. Additionally, the gathering is obtained only in the inelastic component, the elastic component remaining relatively ungathered. Where gathering may be achieved in both layers of a layered composite, as in some stretchable cuffs and waistbands of disposable products of sanitary napkins and diapers, the gathers of one layer are parallel to those of the other layer, both gathers being orthogonal to the direction of stretch.

GB 2284553 (Kimberley-Clark Corporation) describes an incontinence article including a moisture barrier which is gathered along each of its side edges between forward and rearward terminal points and additionally transversely along its back end. The resulting article provides a cupped region. Optionally the longitudinally gathering can be bonded to a liner in the article.

### SUMMARY OF THE INVENTION

It is an object of this invention to provide a gathered, ruffled or puckered layered composite material, having a plurality of layers, wherein at least two of the component layers of the composite are gathered, and wherein the direction of gathering of at least one of the gathered component layers is aparallel to the direction of gathering of an at least one other component layer.

It is a further object of this invention to provide a gathered, layered composite material, having a plurality of layers, wherein the material is a flexible, elastic or relatively nonelastic, fabric, film, foam or combination thereof, wherein at least one of the component layers is reversibly elongatable and thereby tensionable, i.e., stretchable, and is concomitantly reversibly narrowable across its major surface in a direction orthogonal to the direction of tensioning.

It is a further object of this invention to provide a method of making the gathered, layered composite material of this invention.

It is a further object to provide novel absorbent products derived from the gathered, layered composite materials of this invention.

In accordance with the present invention, there has been provided a novel, multi-layered composite material, comprised of at least two layers of a fabric, film, foam or combination thereof, wherein the first layer is a flexible, reversibly elongatable, tensionable material, and which is bonded at spaced apart positions to at least one additional layer of a flexible sheet material, wherein both layers are gathered, the gathers of one layer being aparallel to the gathers of the other layer.

Also provided in accordance with the present invention is a method of forming a gathered, multi-layered composite material, having gathers, ruffles or puckers, comprising the steps of:
first tensioning a first layer of a flexible, reversibly elongatable, tensionable, material such as for example a fabric, film, foam or combination thereof in an amount that is effective to stretch, i.e increase its length beyond its original relaxed untensioned length in the direction of tensioning, and to also results in a narrowing of the width of the first layer to less than its original relaxed, nontensioned width in a direction approximately coplanarly orthogonal to the direction of tensioning;
while in a tensioned state, adhering or otherwise securing the first layer, at discrete spaced apart positions along at least a portion of the first layer, to at least one additional layer of a flexible nontensioned material such for example a fabric, film, foam or combination thereof to form a layered composite material;
and then releasing the tension on the first layer of the composite material to form a gathered, layered composite material; the gathers comprising raised portions of material extending above and below the surface of the composite material, and appearing on both the first layer and the at least one additional layer, the gathers of one layer being aparallel to the gathers of the other layer.

Also provided in accordance with the present invention are novel absorbent products which incorporate the layered composites of this invention, as component parts of these products, including but not limited to diapers, sanitary napkins, incontinence products, as well as bandages, wound dressings, surgical dressings, cushioning and bandages for the treatment of stasis ulcers, surgical drapes, underpads and the like.

### BRIEF DESCRIPTION OF THE DRAWINGS

The above mentioned and other features and objects of this invention, and the manner of attaining them, will become more apparent and the invention itself will be better understood by reference to the following description of embodiments of the invention taken in conjunction with the accompanying drawings, wherein:
Figure 1A is a schematic view of an apparatus for forming the layered composite material of this invention.
Figure 1B is a schematic view of an alternative version of the apparatus shown in Figure 1A for forming the layered composite material of this invention.
Figure 2A is a schematic view of another apparatus for forming the layered composite material of this invention.
Figure 2B is an enlarged plan view of a configuration of bonding stage components used in the apparatus shown in Figure 2A for forming the layered composite material of this invention.
Figure 2C is an enlarged plan view of a alternative configuration of bonding stage components used in the apparatus shown in Figure 2A for forming the layered composite material of this invention.
Figure 2D is a schematic view of an alternative version of the apparatus shown in Figure 2A for forming the layered composite material of this invention.
Figure 2E is an enlarged plan view of a configuration of bonding stage components used in the apparatus shown in Figure 2D for forming the layered composite material of this invention.
Figure 2F is an enlarged plan view of a alternative configuration of bonding stage components used in the apparatus shown in Figure 2D for forming the layered composite material of this invention.
Figure 3 is a plan view, before elongation, and concomitant narrowing orthogonal to the direction of elongation, of a flexible elongatable material used to form the layered composite material of this invention.
Figure 4 is plan view of an elongatable material, after the elongatable material shown in Figure 3 has been elongated and concomitantly narrowed orthogonal to the direction of elongation, intermediate to forming the layered composite material of this invention.
Figure 5 is a plan view of a flexible material used to form the at least one additional layer before it is bonded to the first layer to form the layered composite material of this invention.
Figure 6 is a perspective view of a layered composite material of this invention comprised of the elongatable material, shown in Figure 4, after it has been elongated and concomitantly narrowed orthogonal to the direction of elongation, attached to an at least one additional layer and untensioned to form the layered composite material of this invention.
Figure 7 is a perspective view of another layered composite material of this invention.
Figure 8 is a partial cross section of a perspective view of yet another layered composite material of this invention.
Figure 9 is a perspective view of sanitary napkin made with the layered composite material of this invention.
Figure 10 is a perspective view of another sanitary napkin made with the layered composite material of this invention.
Figure 11 is a perspective view of yet another sanitary napkin made with the layered composite material of this invention.
Figure 12 is a partial cross section of a perspective view of a sanitary napkin comprised of an insert made of the layered composite material of this invention.

### DETAILED DESCRIPTION OF THE INVENTION

The present invention is directed to a novel gathered, layered composite material; and more particularly to a gathered, layered composite material which is comprised of at least two layers of for example a fabric, film, foam or combination thereof, each of the at least two layers being gathered, the gathers of one layer being aparallel to the gathers of the other layer. It is considered an important feature of the present invention that the gathers appear on both the first layer and the at least one additional layer, the gathers of one layer being aparallel to the gathers of the other layer, wherein the aparallel relationship of the gathers of each layer with each other is described as follows:
Each layer is roughly planarly parallel to each other layer. The gathers of each layer have an alignment directionality with each other, in that they are aligned substantially parallel, i.e., in the same direction with each other. The angle of alignment directionality, that the gathers of one layer makes with the alignment directionality of the gathers of the other layer, is preferably greater than 0°, and is most preferably about 90°.
To be noted, is that alignment directionality of the gathers of each layer exists on relatively narrow pieces of the composite material, as may be used, e.g., on a cuff of a sanitary napkin or the waistband of a diaper, as well as on relatively wide pieces of the composite material, as may be used, e.g., on the top layer or the bottom layer of a sanitary napkin. It should also be noted, that alignment directionality is best perceived, without recourse to magnification, on large areas of material , e.g., about one square inch 6.54 cm², than on small areas, such as narrow strips. Such larger areas are particularly useful when one needs to see the alignment directionality of the gathers when the gathers are in the form of, e.g., quilted puckers rather than, e.g., parallel gathers, where the alignment directionality is more readily apparent.

The present invention is also directed to a novel method of making a layered composite material comprising:
Tensioning a first layer, having a length and width, of a flexible material comprised at least in part of a reversibly elongatable, tensionable material such as for example a fabric, film, foam or combination thereof; the tensioning being an amount that is effective to stretch, in approximately the direction of tensioning, the first layer beyond its original relaxed, nontensioned length dimension, and to also cause a narrowing of the first layer, in a direction approximately coplanarly orthogonal to the direction of tensioning, beyond its original relaxed, nontensioned width dimension.

Reversibly elongatable is meant to describe the property of an elongatable material, after being tensioned to stretch the material and the tensioning being relaxed, wherein the material returns to within at least about 86% of its prestretched dimension.

While in a tensioned state, the first layer is adhered or otherwise secured, by bonding means that are at least partially separated from one another, at discrete spaced apart positions along at least a portion of the first layer, to an at least one additional layer of a nontensioned flexible material comprised for example of such materials as a fabric, film, foam or combination thereof, to form a layered composite material comprised of for example a fabric, film, foam or combination thereof. The tension on the first layer of the composite material is then released to form a gathered, layered composite material.

To be further noted, is that the at least one additional layer may undergo some tensioning and concomitant narrowing in the process of forming the composite material of this invention. This tensioning and concomitant narrowing may be tolerated as long as it is insufficient to negate the formation of gathers in the both the first layer and the at least one additional layer, the gathers of one layer being aparallel to the gathers of the other layer.

To be yet further noted, that an important feature of this invention is that the tensioning of the first layer be accompanied by a narrowing of the first layer, in a direction approximately coplanarly orthogonal to the direction of tensioning. This in contrast to the common practice in the art where steps are specifically taken to prevent a narrowing of the material in a direction coplanarly orthogonal to the direction of stretch, when processing an elongatable material that is either purposely placed under tension to stretch the material or is coincidentally under some tension due to differentials in tensions and speeds to which the material is subjected as it is transported and processed from one part of a process another part of the process. Such conventional manufacturing steps include spreading, tensioning and holding the material in a stretched, non-narrowable condition in the orthogonal direction by means such as tentering with pins, clips or belts; and by pulling the material in the orthogonal direction with a ridged cylindrical spreader roll or with a bowed cylindrical roll. The cylindrical spreader roll may for example have the ridges not connected to each other, or connected as in a continuous spiral around the cylindrical spreader roll, or combinations thereof. The ridges or spiral preferably have high friction surfaces, e.g., of soft rubber. The ridges also are preferably disposed, at acute angles, with respect to either side of the side of center and the direction of rotation of the cylindrical spreader roll. The surface of the bowed cylindrical roll, over which the material to be maintained in a non-narrowable condition passes, is preferably convex. The surface of the bowed cylindrical roll is also preferably made of a high friction material, e.g., of soft rubber. Additionally the material may be held in a non-narrowable condition by means of pinch or nip rolls that are applied to the material as it travels over the various cylindrical rolls described above.

Materials useful for the both the first layer and the at least one additional layer of this invention are required to be flexible in that they are capable of bending or folding so as to gatherable. Fabrics, films, foams or combinations thereof useful for both layers of this invention may also include laminates of these materials.

The first layer of this invention may be chosen from materials such as: elastic reversibly elongatable or partially reversibly elongatable, tensionable fabrics, films, foams or combinations thereof; and nonelastic reversibly elongatable or partially reversibly elongatable, tensionable fabrics, films, foams or combinations thereof. All the materials from which the first layer of this invention may be made, on being tensioned and thereby elongated, should be selected from those being concomitantly capable of undergoing a reversible narrowing in a direction approximately coplanarly orthogonal to the direction of tensioning. Therefore, the first layer of this invention must be elongated to such an extent and concomitantly narrowed orthogonal to the direction of tensioning to such an extent, to enable the tensioning and the narrowing to effect gathering of both the at least one additional layer and the first layer, respectively.

The at least one additional layer may be chosen from flexible materials such as: elastic or nonelastic reversibly elongatable or partially reversibly elongatable, tensionable fabrics, films, foams or combinations thereof; nonreversibly elongatable, tensionable fabrics, films, foams or combinations thereof; and nonelongatable fabrics, films, foams or combinations thereof.

Elastic materials are those capable of rapidly returning to approximately their initial dimensions and shape after being substantially deformed by a deforming force, usually a weak force, and removal of such force.

Nonelastic materials are those that are capable of being tensioned and thereby stretched without exceeding their yield point and then returning essentially to their original dimensions and shape when the tensioning force is removed. Such preferred nonelastic films fabrics and foams are those capable of being tensioned, thereby being stretched, to at least 1% greater than their original length without reaching their yield point, the yield point being the first point on a stress-strain curve at which an increase in strain (here the stretch or elongation), occurs without an increase in stress (here the tensioning force), i.e., where the material has been stretched but no longer returns to its original length. It is preferred that the nonelastic fabrics, films or foams be capable of being stretched, without reaching their yield point, from 1% to at least about 60% greater than their original length and preferably between 5% to about 35% greater than their original length.

It should be noted that elastic or nonelastic materials which are only partially reversibly elongatable may be used in this invention, as long as sufficient elongation reversibility and shortening reversibility resides in the materials to create the gathers of this invention.

The ability of a fabric, film or foam to be elongated is required at least in one direction. The at least one direction may be in the machine direction, the cross machine direction and directions therebetween; with the proviso that the material be concomitantly narrowed in a direction approximately coplanarly orthogonal to the direction in which the material is elongated.

Fabrics, films and foams that are capable of being biaxially elongated are also useful in this invention. Thus, the reversibly elongatable, tensionable fabrics, films and foams useful in this invention may be capable of being tensioned in a single direction, in two directions, or in a plurality of directions, e.g., where the film or fabric is stretched in both the X and Y directions relative to its surface, as well as in its upward or Z direction, either in a specific zone area or over its entire surface. The foregoing notwithstanding, it is important in the practice of this invention, that biaxially elongatable materials be tensioned in a first direction, e.g., the machine direction, and also be allowed to narrow in a second direction, e.g., the crossmachine direction, that is coplanarly orthogonal to the first direction. If, however, biaxially elongatable materials are used and are also tensioned in the second orthogonal direction, it is required that they be tensioned in the second direction to a degree that can be surpassed by the potential of the material to recover by expanding; and thereby be capable of expanding coplanarly orthogonal to direction of tensioning, from the narrowing it experienced when it was in tension.

Reversibly elongatable, tensionable materials that are useful for the first layer and the at least one additional layer are well known in the art, and are represented either by elastic films, fabrics and foams and by nonelastic films, fabrics and foams that are capable of being tensioned and are able to return at least in part to their original dimensions and shape when the tensioning force is removed. Materials useful for the at least one additional layer may also include nonelongatable films, fabrics or foams.

Suitable films useful for the first layer and the at least one additional layer of this invention may be chosen from those that are continuous, i.e., nonperforate, or from those that are perforate. The perforate films include those: having apertures that are either essentially in the plane of the film; having apertures that are three dimensional in the sense that the defining walls of the apertures extend beyond the plane of the film; and perforate films having combinations of in the plane apertures and apertures that are three dimensional. Films are also meant to include those that are embossed. The embossments include those that are large enough to be seen by eye or to be felt by touch; and also include those that are not easily detected by eye or by touch, e.g., microembossments that may lend a matte finish to the film and may also lend a fabric feel to the film. Elasticity, elongability and the ability to be narrowed, for films, will generally, but not without exception, increase with reduced thickness, increased plasticization, increased number of apertures and increased area occupied by the apertures. Of course, the chemical nature, molecular weight and degree of crosslinking, plasticizer concentration and other factors will also dictate the elasticity and elongatability of the film. Films that are approximately less than or equal to 10 millimeters thick are preferred; with films that are approximately equal to or less than 3 millimeters thick being more preferred.

Suitable fabrics useful for the first layer and the at least one additional layer of this invention may include flexible paper products such as tissues and flexible fabrics that are woven, knitted or nonwoven. The tissues and woven and knitted fabrics may include those that are made by any of the several processes that are known in the arts of paper making, weaving and knitting, respectively, and yield the various tissue and fabric structures and designs that are known in these arts. Nonwovens, however, are particularly preferred for their low cost and the wide range of properties that can be imparted to them by the processes of forming and finishing and by the materials from which they are made. Suitable nonwoven fabrics, as defined by the processes by which they are made, may include wetlaid, airlaid, spunbond, meltblown, chemically bonded, thermally bonded, hydroentangled nonwoven fabrics and nonwoven fabrics resulting from combinations of such processes. Nonwovens are more likely to be elongatable if they are of low basis weight, bonded lightly or not at all, e.g., hydroentangled, meltblown, and lightly bonded spunbond, thermally bonded and chemically bonded nonwovens. Chemically bonded nonwovens are more likely to be elongatable if they contain elastic and elongatable fibers and contain elastic or frangible binders. The direction of the fibers, e.g., random as resulting from airlaying, or unidirectional, as resulting from carding, will also determine the direction in which the nonwoven will most easily elongate and the ease with which it will elongate in each direction. Fabrics of basis weight about equal to or less than 100 grams/square meter are preferred; with fabrics of basis weight about equal to or less than 20 grams/square meter being more preferred.

Suitable foams useful for the first layer and the at least one additional layer of this invention may be chosen from flexible and elongatable reticulated and nonreticulated foams. Such foams may be made of, but not restricted to, polyester polyurethanes, polyether polyurethanes and polyethylene. The degree of elasticity will depend, in varying degrees and parts, on properties such as the thickness, durometer and degree of plasticization of the foam and the degree to which the foam is reticulated, i.e., whether the foam cells are in communication with one another or are isolated from one another by the cell walls of the foam. In general, but not without exception, elasticity and elongatability increase with decrease in thickness and durometer and with increase in plasticization and degree of reticulation. Of course, the chemical nature, molecular weight and degree of crosslinking, plasticizer concentration and other factors will also dictate the elasticity and elongatability of the foam. Foams less than or equal to 6.35 millimeters thick, are preferred; with foams that are approximately equal to or less than 3.175 millimeters thick being more preferred.

Suitable elastic films and fabrics are those made of elastomeric materials such as natural and synthetic rubbers, elastomeric polyurethanes (ESTANE from B. F. Goodrich & Co.), elastomeric polyamides (PEBAX from Atochem Co.) and elastomeric polyesters (HYTREL from E. I. DuPont de Nemours & Co.), styrene butadiene styrene block copolymers, styrene ethylene butylene styrene block copolymers, styrene isoprene styrene block copolymers (examples of these block copolymers are those having the trademark KRATON from Shell Chemical Co.), noncrosslinked ethylene vinyl acetate copolymers, ultralow density polyethylene, blends, coextruded laminates, layered laminates of such materials, and combinations thereof.

Suitable elongatable nonelastic fabrics or films for use in this invention include, but are not limited to, those made of materials such as polyethylene, preferably low and medium density polyethylene, polypropylene, noncrosslinked ethylene vinyl acetate copolymers, polyamides such as nylon 6 and nylon 6,6, polyesters, polyvinylidene chloride, polyvinyl chloride, polyester polyurethanes, polyether polyurethanes, and blends, coextruded laminates, layered laminates of such materials, and combinations thereof.

A preferred elongatable, nonelastic film for use in this invention is polyethylene film. The film may be nonperforate if for example in a sanitary napkin it is used as a barrier or as a component material of the cuffs, wings or tabs of the sanitary napkin. The preferred elongatable nonelastic film may be perforate if for example it is used as a cover or fluid transfer layer or as a component material of the cuffs, wings or tabs. Similar choices of perforate or nonperforate materials, their use and placement in each product, may be made in the manufacture of other products such as, but not limited to, sanitary absorbent products including diapers, incontinence products, as well as bandages, wound dressings, surgical dressings, cushioning and bandages for the treatment of stasis ulcers, surgical drapes, underpads and the like.

Suitable fibers and filaments for the fabrics useful for this invention may be chosen for the degree of elongatability and elasticity desired. Since filaments may be thought of as long fibers, the fibers and filaments referred to herein will be referred to as fibers and thereby deemed to include both fibers and filaments. Fibers such as woodpulp, cotton, rayon, wool, linen, silk, ramie and jute are relatively nonelongatable and nonelastic. Relatively more elongatable and elastic are synthetic fibers such as polyethylene, polypropylene, polyester, polyester polyurethane, polyether polyurethane; and bicomponent fibers such as of polyethylene and polypropylene, polyethylene and polyester, polypropylene and polyester and low and high melting polyesters. In addition, the fiber denier, cross-section, degree of crystallinity will also impact its elongatability and elasticity. The degree that the fabric permits movement of one fiber with respect to another, and the flexibility, elongatability and elasticity of any binder that is used, whether it is an adhesive or thermoplastic binder, and whether the binder itself is elastic, will also dictate to some degree the elongatability and elasticity of the fabric.

The gathered, composite of this invention may optionally comprise a laminated structure made of more than two plies. The following examples of composites comprising more than two plies are presented without meaning to be restricted by such examples. For example, a three ply laminated structure may be made wherein an elongatable elastic or nonelastic fabric, film, foam or combination thereof is first tensioned, and to opposite sides thereof is bonded one each of two additional nontensioned layers of fabric, film or foam; or one of the two nontensioned additional layers may be adhered to the tensioned layer followed by sequentially adhering the second of the two additional nontensioned layers to the first additional nontensioned layer; after which the tensioned layer is relaxed to form a gathered, layered composite. Alternatively, two layers of tensioned elongatable fabric, film or foam may be adhered or secured, alternately one on either side or both to one side of a nontensioned additional layer; after which the tensioned layers are allowed to relax to form a gathered layered composite. Additional layers of tensioned elongatable and nontensioned fabrics, films and foams may be similarly laminated. One may substitute, for any layer of the composite, a component layer that does not become gathered, by means such as attaching it to a layer or component after the tensioned composite or tensioned layer has been untensioned; or by using a layer made of a tensioned material that is not reversibly elongatable in the direction of tensioning and reversibly narrowable orthogonal to the direction of tensioning.

The elongatable elastic or nonelastic fabric, film or foam, and the at least one additional layer of fabric, film or foam may be adhered or otherwise secured to each other by any conventional techniques which are known for securing two or more layers of flexible sheet materials together and includes, but is not limited to, the use of adhesives, heat sealing, ultrasonic bonding, radiofrequency sealing, mechanical crimping, cold flow pressure sealing, and the like, and combinations thereof. Adhesive may be applied as a hotmelt, for example, by spraying or through separate orifices or through a slotted orifice which has spacers to separate the exiting adhesive into spaced apart streams. Each coating stream or spray may be discontinuous or continuous. Adhesive may also be applied by screen printing or by transfer coating. Hotmelt adhesives are preferred over solvent based adhesives for speed of application and for the elimination of the need to remove byproducts such as solvents and components of curing agents. The adhesive may be applied to either or both the elongatable elastic or nonelastic fabric, film or foam and the at least one additional layer of fabric, film or foam. However, if adhesive is used, it is preferred, for ease and accuracy of application, that it be applied to the at least one additional layer of fabric, film or foam, since the at least one additional layer is not being tensioned or is tensioned to a lesser degree than the first layer of material, and is thus more easily kept laying flat during the process of manufacture, albeit that it may be inherently three dimensional by virtue of being for example embossed or by having three dimensional apertures. Mechanically applied methods of sealing such as heat sealing, ultrasonic bonding, radiofrequency sealing, mechanical crimping, cold flow pressure sealing, may be accomplished with a heated roll or stamping plate that has a patterned surface to which the appropriate energy, i.e., heat, ultrasound, radiofrequency, pressure or combinations thereof, is applied.

The elongatable elastic or nonelastic fabric, film or foam, and the at least one additional layer of fabric, film or foam may be bonded to each other in any manner or pattern of adherement or securement that provides at least partially spaced apart zones of bonding, i.e., the zones of adherement or securement are separated from one another by zones of non adherement or securement. The zones of adherement or securement should be wide enough to retain the integrity of the bonding between the layers of the composite material during manufacture or use. In practice, this width has been found to be at least about 0.397 mm (0.016 in.) and preferably at least about 0.793 mm (0.031 in.). However, such zones should not be so wide as to severely reduce the areas of material that is gathered or to compromise the ability of the material to be gathered. In practice, the maximum width of the zones of adherement or securement has been found to be about 12.7 mm (0.5 in.) and preferably about 9.525 mm (0.375 in.).

Such manner and pattern of adherement or securement provide areas where the adhered fabrics, films and foams can relax to form gathers. Examples of the elements of such patterns of zones of adherement or attachment are spaced apart straight or curved lines or bars, where the lines or bars may be continuous, or may be made up of a series of discontinuous and/or continuous dots, dashes, chevrons, circles or other geometric shapes, swirls and zigzags and the like and combinations thereof. Examples of curved patterns are undulating sinusoidal lines or bars.

The zones of non-adherement or spacing between the elements of the zones of adherement may be range from about (0.0625 in.) 1.588 mm to 12.7 mm (0.5 in.) and may preferably be between (0.125 in.) 3.175 mm and (0.375 in.) 9.525 mm. It should be noted that the spacing used will be dictated by both the degree of gathering, the amount of material desired in the gathers, as well as the loft of the materials used. Thus closer spacing between the elements of the zones of aherement or securement may be tolerated when the material is relatively thin and flexible. Thicker material will require the use of wider spacing between the elements of the zones of adherement or securement.

The general direction of the pattern of the zones of adherement or attachment, if it is nonrandom such as e.g. discrete lines of adhesive in a spaced apart pattern, may be at any angle to the direction in which the elongatable elastic or nonelastic fabric, film or foam is tensioned. When the elongatable elastic or nonelastic fabric is relaxed the puckers will be seen to reflect the pattern of the elements of the zones of adherement or securement, e.g., discrete points or lines wherein the lines may be continuous, discontinuous, regular, random, straight or curved, and wherein the angle that the pattern of elements of the zones makes with the direction in which the elongatable elastic fabric, film or foam has been stretched, i.e parallel, orthoganal or any angle in between. The resulting gathers will then be seen to vary, for example, from a series of parallel ruffles or ridges to a pattern of quilted puckers of different sizes, shapes and outlines. It has been discovered that after tensioning is relaxed, that the gathers can be discerned to have a pattern of directionality, e.g., a series of parallel ruffles or ridges, then the pattern of gathers along the plane of one layer, e.g., the elongatable elastic fabric, film or foam, is approximately orthogonal to the pattern of gathers in the at least one other layer along the plane of that other layer.

A preferred film useful in this invention is for example an elongatable or nonelastic nonperforate or perforate polyethylene or polypropylene film. Examples of such perforate nonelastic films are described in the following Patent Applications, and commonly assigned: to Turi et al, "Textilelike Apertured Plastic Films", Canadian Patent Application 2,130,176; "Method of Forming Improved Apertured Films, Resultant Apertured Films and Absorbent Products Incorporating Resultant Apertured Films"; to James et al, US Patent Application No. 08/523,112 (corresponding to EP 96.931423.6); and to Burwell et al, "Apertured Film Having improved Fluid Distribution Properties, Method of Forming Same and Absorbent Products Incorporating Same", US Patent Application No. 08/522,600 (corresponding to EP 96.929077.4); and "Absorbent Products", US Patent Application No. 08/522,722.

Other examples of perforate films are described in US 3,054,148 to Zimmerli, US 4,690,679 and US 4,806,411 to Mattingly et al, US 4,859,519 to Cabe et al, US 3,929,135 to Thomson et al, US 4,324,246 to Mullane and European Patent Application 0,304,617 to Kao Corporation.

The perforate film is tensioned and an additional permeable layer of fabric, film or foam is adhered thereto, the zones of adherement are separated from one another by zones of non adherement or nonsecurement. Examples of such additional permeable layers of fabric, film, or foam are a nonwoven fabric, tissue, perforated film and reticulated foam. The tension on the elastic or nonelastic fabric, film or foam is then released to form a gathered three-dimensional fluid pervious composite.

The gathered layered composite materials formed in accordance with such preferred films, when incorporated into an absorbent product, provide soft, comfortable gathered textures that have less contact with the body than do flat structures. The composites also provide surprisingly enhanced fluid transfer properties as well as unique structural and spatial attributes, and have been found useful in the manufacture of various products including, but not limited to, sanitary absorbent products such as diapers, sanitary napkins, incontinence products, as well as bandages, wound dressings, surgical dressings, cushioning and bandages for the treatment of stasis ulcers, surgical drapes, underpads and the like. Some structure benefits, as examples, are the properties of preventing wet collapse and providing compression and crush resistance to such products during use. This preferred embodiment of the perforate layered composite material of this invention has also been found to be particularly suitable for use in the absorbent products, as cover layers and as intervening fluid transfer layers. When at least a part of the layered composite material of this invention is nonperforate, it has been found useful in these products as barrier layers and as covers for cuffs, wings or tabs. When the layered composite material has at least one perforate component layer, it has been found useful in these products as intervening fluid transfer layers and as containment structures for ingredients such as deodorants, fragrances, medicaments and superabsorbents. When the layered composite material is comprised completely of either perforate or nonperforate component layers or has both perforate and nonperforate component layers, it has been found particularly useful as deformation and crush resistant structures. Examples of lateral and vertically wicking intervening fluid transfer layers are described in "Absorbent Articles with Plates", US Patent Application Serial No. 08/220,073 to Hsieh et al, and commonly assigned.

In accordance with this aspect of this invention, the gathered composite material is incorporated into sanitary napkins, adult incontinence products, bandages, and the like, there has now been provided an absorbent product having a top fluid permeable layer, a bottom fluid impermeable layer, and an absorbent core therebetween, wherein the top fluid permeable layer comprises a perforate polyethylene film which is first tensioned to stretch the film to a length which is at least 101% of its original nontensioned length, the film concomitantly undergoing narrowing in a direction orthogonal to the direction of tensioning; adhering a second fluid pervious fabric, film or foam to the perforate polyethylene film by adhering or securing means that are at least partially separated from one another, at discrete spaced apart positions along at least a portion of the tensioned perforate film; and then releasing the tension on the perforate polyethylene film to yield a product with a gathered top fluid permeable layer. This gathered, layered composite material may comprise the bottom, garment facing, fluid impermeable layer, to prevent leakage from the bottom and sides of the product. In such uses, it is preferred that either or both the first elongatable fabric, film or foam and the second fabric, film or foam be fluid impervious. The sanitary napkin may optionally have wings or tabs to fix the napkin to the undergarment. The gathered composite material of this invention may comprise all or part of the wings or tabs. The napkin may also optionally contain embossments or deep channels, to raise the absorbent center and thereby the fluid pervious top layer to contact the body, and to channel fluids longitudinally to prevent side leakage. The absorbent core of the napkin may optionally contain a gathered composite material of this invention in the form of lateral and vertically wicking intervening fluid transfer layers and/or as containment structures for ingredients such as deodorants, fragrances and superabsorbents.

In a another embodiment of this invention, there has been provided a sanitary napkin having a fluid pervious top layer, a fluid impervious bottom layer, and an absorbent core therebetween, and having gathered longitudinal edges, which may optionally form upstanding sidewalls to inhibit fluid leakage across the lateral sides of the sanitary napkin. The gathered edges may be formed by tensioning a first elongatable fabric, film or foam, and while in its tensioned state, the elongatable fabric, film or foam is concomitantly narrowed in a direction orthogonal to the direction of tensioning; adhering a second fabric, film or foam to the elongatable fabric or film by adhering or securing means that are at least partially separated from one another, at discrete spaced apart positions along at least a portion of the tensioned elongatable fabric or film, and then releasing the tension on the elongatable fabric, film or foam to yield a product with a gathered composite material wherein both layers are gathered, the gathers of one layer being aparallel to the gathers of the other layer. This gathered composite material may then be adhered or secured along side edges of a sanitary napkin. The gathered composite material, before being adhered or secured along the side edges of a sanitary napkin, may itself be tensioned, the tension being released after it is adhered or secured to the side edges, thereby raising and providing curvature to the gathered edges, the upstanding sidewalls or the napkin itself. The gathered composite material may also comprise the bottom, garment facing, fluid impermeable layer, to prevent leakage from the bottom and sides of the product.

The sanitary napkin prepared in accordance with this aspect of the invention provides the sanitary napkin with soft gathered edges and/or upstanding side walls which are not easily deformed and which, therefore, act as substantial liquid barriers to prevent side leakage. In such uses, it is preferred that either or both the first elongatable fabric, film or foam and the second fabric, film or foam be fluid impervious. The sanitary napkin may optionally have wings or tabs to fix the napkin to the undergarment. The gathered, layered composite material of this invention may form all or part of the wings or tabs. The napkin may also optionally contain embossments or deep channels, to raise the absorbent center and thereby the fluid pervious top layer to contact the body, and to channel fluids longitudinally to prevent side leakage.

Without restricting the invention in any way, the following are examples of processes, which have been found to be suitable for making the gathered, layered composite material of this invention.

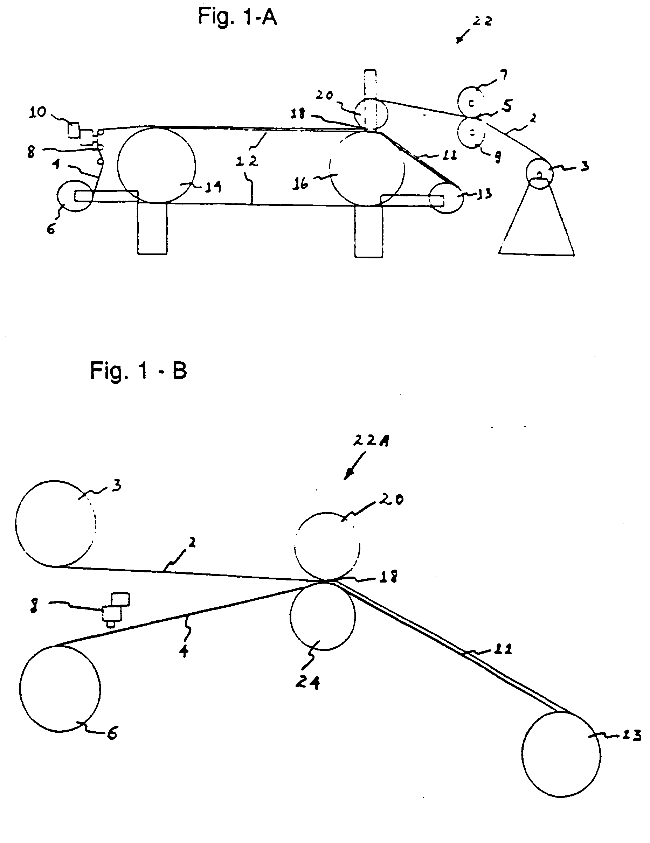

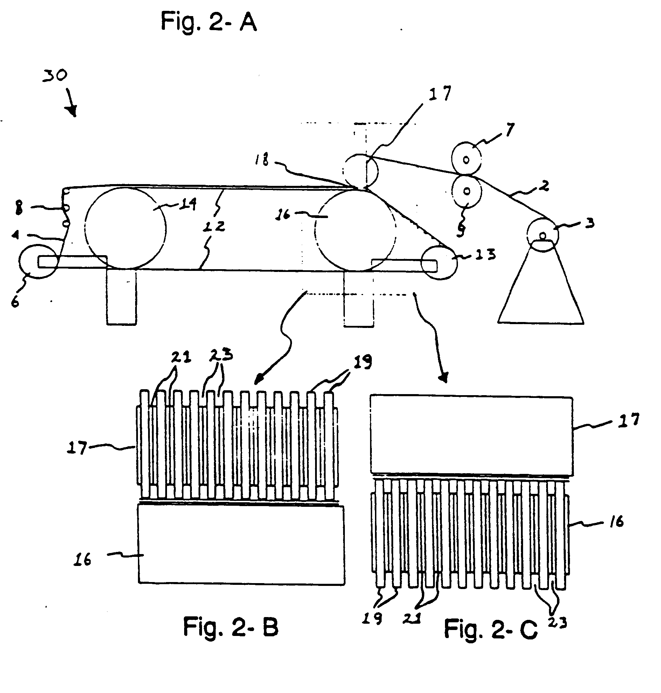

Figures 1A and 1B are schematic views of an apparatus **22**, and an alternative apparatus **22A** to apparatus **22**, respectively, for forming the layered composite material of this invention. Analogous or similar elements in each Figure are given the same designating numbers. Referring now to Figure 1A, the process requires at least a first layer **2** of a flexible, substantially reversibly elongatable material, such as a fabric, film or foam, and a second layer **4** of a flexible material, such as a fabric, film or foam. The two layers may be of different materials or of the same material. Second layer **4** is unwound from a supply roll **6** and passed under and over a series of guide rolls **8**. During the passage through series of guide rolls **8**, second layer **4** is subjected to coating by hotmelt adhesive that is fed from a slot coater **10**, wherein the slot is divided into open and closed zones by a series of spacers, the hotmelt adhesive being thereby separated into discrete streams that impact and thereby coat second layer **4** with a spaced apart pattern, e.g., at least two parallel lines, of adhesive. Details of the slot coater, the divided slot, the hotmelt adhesive and the coating pattern, being known in the art, are not shown in Figure 1a. It should be noted that the pattern of adhesive, here the at least two parallel lines, on second layer **4** may be at any angle, from 0 to 90 degrees to the machine direction; and that the spaced apart pattern may be of a variety of designs, not necessarily parallel lines, as is discussed earlier in the section, "Detailed Description of the Invention". Second layer **4** is led onto and is supported by a belt **12** that is rotated belt supporting rolls **14, 16.** Second layer **4** is further led through a nip **18** between belt **12** on belt supporting roll **16**, and a drive roll **20**; where, in nip **18**, second layer **4** is laminated to a tensioned, elongated and concomitantly orthogonally narrowed first layer **2** that is being brought into nip **18** on top of, and coincidingly with, second layer **4**.

First layer **2** is unwound from a supply roll **3** and led through a nip **5** between a drive roll **7** and a pressure roll **9**, with drive roll **7** being rotated at a slower speed than that of drive roll **20**. The tensioned and concomitantly narrowed first layer **2** is led around drive roll **20** and through nip **18** between drive roll **20** and incoming second layer **4** where it is laminated to second layer **4** as described above.

Alternatively, as in apparatus **22A** in Figure 1B, belt **12** may be eliminated, with both first layer **2** and second layer **4** being led directly through nip **18** between drive roll **20** and pressure roll **24**, with drive roll **20** rotating at a faster speed than supply roll **3**; thereby causing first layer **2** to be tensioned and thereby stretched as it passes through nip **18** to be laminated to and second layer **4**.

Since, in apparatus **22**, and apparatus **22A**, drive roll **20** is rotating faster than drive roll **7**, and supply roll 3 respectively, first layer **2** is tensioned and thereby stretched in the machine direction and concomitantly narrowed in the cross machine direction.

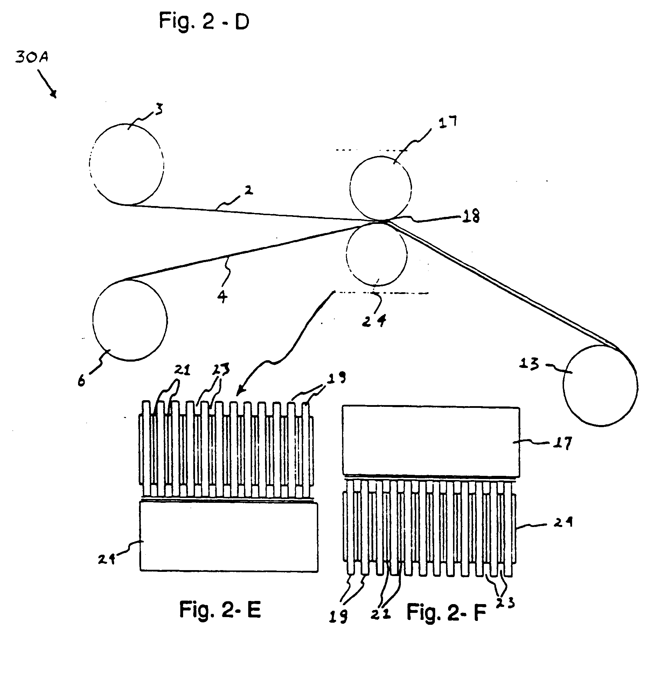

In both Figures 1A and 2A, after bonding the concomitantly tensioned and narrowed first layer **2** to second layer **4**, both the tensioning and concomitant narrowing of first layer **2** are permitted to relax, thereby allowing the layered material leaving nip **18** to form a three dimensional, layered composite material **11**, wherein both first layer **2** and second layer **4** are gathered, the gathers of one layer being aparallel to the gathers of the other layer. Composite material **11** is wound on to a takeup roll **13** for further use.

Figures 2A and 2D are schematic views of another apparatus **30**, and alternatives to apparatus **30**, and an alternative apparatus **30A** to apparatus **30**, respectively, for forming the layered composite material of this invention. Figures 2B and 2C are enlarged plan views of alternative configurations of the bonding stage components shown in Figure 2A. Figures 2E and 2F are enlarged plan views of analogous alternative configurations of the bonding stage components shown in Figure 2D. Analogous or similar elements to those in Figures 1A and 1B are given the same designating numbers in Figures 2A to 2F. Referring now to Figure 2A, the process requires at least a first layer **2** of a flexible, substantially reversibly elongatable material, such as a fabric, film or foam, and a second layer **4** of a flexible material such as a fabric, film or foam. The two layers may be of different materials or of the same material. Second layer **4** is unwound from a supply roll **6** and passed under and over a series of guide rolls **8**. Second layer **4** is led onto and is supported by a belt **12** that is rotated by belt supporting rolls **14, 16**. Second layer **4** is led through a nip **18,** between belt **12** on belt supporting roll **16,** and a drive/heat sealing roll **17;** where, in nip **18,** second layer **4** is heat laminated in a spaced apart pattern to a tensioned, elongated and concomitantly orthogonally narrowed first layer **2** that is being brought into nip **18** on top of, and coincidingly with, second layer **4**. An example of a drive/heat sealing roll **17** is shown in Figure 2B in plan in enlarged detail, wherein drive/heat sealing roll **17** is shown to contain, for example, at least two spaced apart raised heated ridges **19**, ridges **19** being spaced apart by spacers **21** that are at a level lower than ridges **19**, thereby forming grooves **23** between ridges **19.**

Belt supporting roll **16** may be smooth surfaced or it may have ridges that either meet with or interdigitate ridges **19** on drive/heat sealing roll **17**.

First layer **2** is unwound from a supply roll **3** and led through a nip between a first drive roll **7** and a pressure roll **9**, with drive roll **7** being rotated at a slower speed than that of drive/heat sealing roll **17**. Since drive/heat sealing roll **17** is rotating faster than drive roll **7**, first layer **2** is tensioned and thereby stretched in the machine direction and concomitantly narrowed in the cross machine direction. The tensioned and concomitantly narrowed first layer **2** is led around a drive/heat sealing roll **17** and through nip **18** between drive/heat sealing roll **17** and incoming second layer **4** where it is laminated to the second layer as described above. Alternatively, with continued reference to Figure 2B, drive/heat sealing roll **17** may be unheated, belt supporting roll **16** being heated, thereby having the heat coming from belt supporting roll **16**, through belt **12** and second layer **4** to heat seal and thereby laminate the two layers **2, 4** together. Yet alternatively, as in Figure 2C, belt supporting roll **16** may be heated and contain, for example, at least two spaced apart raised heated ridges **19**, ridges **19** being spaced apart by spacers **21** that are at a level lower than ridges **19**, thereby forming grooves **23** between ridges **19**. In such a case, drive roll **17** may unheated and either be smooth surfaced or have ridges that either meet with or interdigitate ridges **19** on belt supporting roll **16**. It should be noted that belt supporting roll **16** and drive roll **17** may optionally both be heated.

Alternatively, as in apparatus **30A** in Figure 2D, belt **12** may be eliminated, with both first layer **2** and second layer **4** being led directly through nip **18** between drive roll **20** and pressure roll **24**, with drive roll **20** rotating at a faster speed than supply roll **3**; thereby causing first layer **2** to be tensioned and thereby stretched in the machine direction and concomitantly narrowed in the cross machine direction, as it passes through nip **18** to be laminated to and second layer **4**. With reference to Figure 2E, drive roll is heated and contains for example, at least two spaced apart raised heated ridges **19**, ridges **19** being spaced apart by spacers **21** that are at a level lower than ridges **19**, thereby forming grooves **23** between ridges **19**. Pressure roll **24** may be smooth surfaced or it may have ridges that either meet with or interdigitate ridges **19** on drive roll **20**.

Alternatively, with reference to Figure 2F, drive roll **17** may be unheated, pressure roll **24** being heated, and containing, for example, at least two spaced apart raised heated ridges **19**, ridges **19** being spaced apart by spacers **21** that are at a level lower than ridges **19**, thereby forming grooves **23** between ridges **19**. In such a case, drive roll **17** may unheated and either be smooth surfaced or have ridges that either meet with or interdigitate ridges **19** on pressure roll **24**. It should be noted that pressure roll **24** and drive roll **17** may optionally both be heated.

After heat sealing the concomitantly tensioned and narrowed first layer to the second layer, both the tensioning and concomitant narrowing of the first layer are permitted to relax, thereby allowing the layered material leaving nip **18** to form a three dimensional, layered composite material **11**, wherein both first layer **2** and second layer **4** are gathered, the gathers of one layer being aparallel to the gathers of the other layer. Composite material **11** is wound on to a takeup roll **13** for further use.

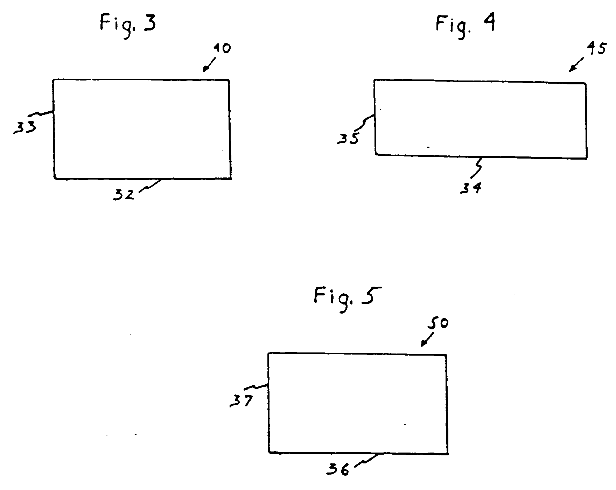

Figure 3 is a plan view, before elongation, and concomitant narrowing orthogonal to the direction of elongation, of a flexible elongatable material **40** used to form the first layer of the layered composite material of this invention. Material **40** is described in Figure 3 to have a machine direction dimension **32** and a crossmachine direction dimension **33**, being the respective directions in which the first layer will be tensioned and thereby elongated and be concomitantly narrowed, in the process of making the layered composite of this invention.

Figure 4 is plan view of an elongatable material **45**, after the elongatable material shown in Figure 3, has been elongated and concomitantly narrowed orthogonal to the direction of elongation, intermediate to forming the first layer of the layered composite material of this invention. Material **45** is described in Figure 4 to now have a machine direction dimension **34** and a crossmachine direction dimension **35**, being the respective directions in which the first layer has been tensioned and thereby elongated, wherein the dimensions **34** and **35** are respectively longer and shorter than dimensions **32** and **33** shown in Figure 3.

Figure 5 is a plan view of a flexible material **50** used to form the at least one additional layer before it is bonded to the first layer to form the layered composite material of this invention. Material **50** is described in Figure 5 to have a machine direction dimension **36** and a crossmachine direction dimension **37**, being the respective directions in which the at least one additional layer will aligned with the first layer in the process of making the layered composite of this invention.

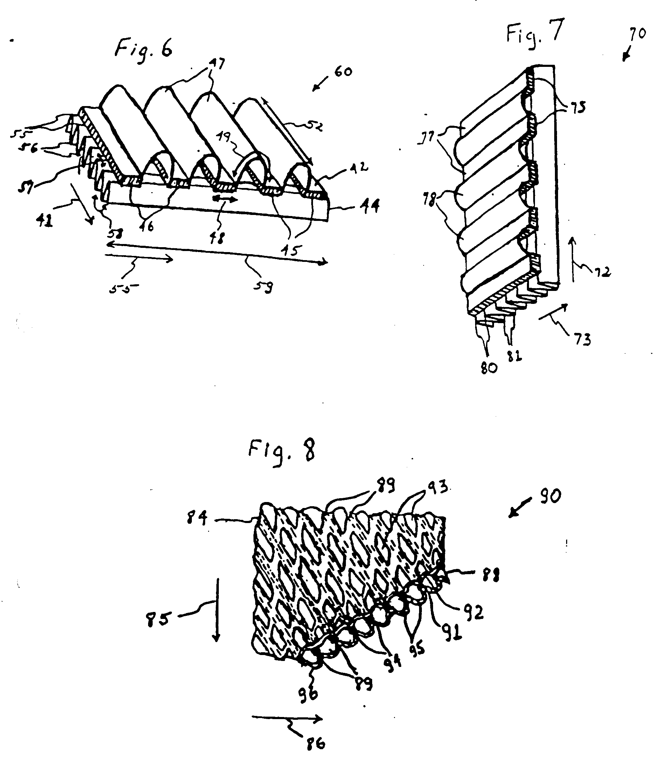

Figure 6 is a perspective view of a gathered layered composite material **60** of this invention comprising the elongatable material, shown in Figure 4, now shown as the first layer component **42** of gathered layered composite material **60** of this invention, after first layer **42** has been elongated in the machine direction **41**, and concomitantly narrowed orthogonal to the direction of elongation, i.e., in the crossmachine direction **55**, and attached to an at least one additional layer **44** by spaced apart lines of adhesive **45**, wherein the spaced apart lines of adhesive are essentially parallel to the machine direction **41**; and the tensioning and concomitant narrowing is relaxed to form layered composite material **60** of this invention, wherein both layers **42, 44** are gathered, the gathers of one layer being aparallel to the gathers of the other layer.

First layer component **42** comprises bonded regions **46** and nonbonded regions **47**, having respectively linear cross section dimensions **48, 49** and a machine direction dimension **52**. On sequentially comparing dimensions shown in Figure 6 with the analogous dimensions shown in Figure 3, the sum of linear cross section dimensions **48, 49** are essentially yet approximately equal to crossmachine direction dimension **32** shown in Figure 3; and machine direction dimension **52** is essentially yet approximately equal to machine direction dimension **33**, also shown in Figure 3.

At least one additional layer **44** comprises bonded regions **55** and nonbonded regions **56**, having respectively linear cross section dimensions **57, 58** and crossmachine direction dimension **59**. On sequentially comparing dimensions shown in Figure 6 with the analogous dimensions shown in Figure 5, the sum of linear cross section dimensions **57** and **58** are essentially yet approximately equal to machine direction dimension **37** shown in Figure 5 and crossmachine section dimension **59** is essentially but approximately equal to crossmachine direction dimension **36** shown in Figure 5.

Figure 7 is a perspective view of another gathered, layered composite material **70** of this invention. Composite material **70** comprises a first layer component **71** that has been elongated in the machine direction **72** and concomitantly narrowed orthogonal to the direction of elongation, i.e., in the crossmachine direction **73** and attached to an at least one additional layer **74** by spaced apart lines of adhesive **75**, wherein the spaced apart lines of adhesive are essentially parallel to cross machine direction **73**; and the tensioning and concomitant narrowing is relaxed to form the layered composite material **70** of this invention, wherein both layers **71, 74** are gathered, the gathers of one layer being aparallel to the gathers of the other layer.

First layer component **71** comprises bonded regions **77** and nonbonded regions **78**, which regions together define the gathers **79** of first layer component **71**. At least one additional layer **74** comprises bonded regions **80** and nonbonded regions **81**, which regions together define the gathers **82** of at least one additional layer component **74**. Analogous comments may be made about the relationship of the bonded and nonbonded regions of the gathered regions of this layered composite with respect to the dimensions of both the unperturbed starting first layer and the at least one additional layer.

Figure 8 is a partial cross section perspective view of yet another gathered, layered composite material **90** of this invention. Composite material **90** comprises a first layer component **84** that has been elongated in the machine direction **85** and concomitantly narrowed orthogonal to the direction of elongation, i.e., in the crossmachine direction **86** and attached to an at least one additional layer **88** by spaced apart lines of adhesive **89**, the adhesive lines being shown predominantly in phantom, wherein the spaced apart lines of adhesive are essentially not parallel to either machine direction **85** or to crossmachine direction **86**, but are at some nonparallel angle to either direction, for example, 45 degrees to machine direction **85**; and the tensioning and concomitant shortening is relaxed to form the layered composite material **90** of this invention; wherein both layers **84, 88** are gathered, the gathers being in the form of quilted rather than parallel puckers, and the gathers of one layer being aparallel to the gathers of the other layer.

First layer component **84** comprises bonded regions **91** and unbonded regions **92**, which regions together define the gathers **93** of first layer component **84**. At least one additional layer **88** comprises bonded regions **94** and nonbonded regions **95**, regions **94, 95**, which regions together define the gathers **96** of at least one additional layer component **88**. Analogous comments may be made about the relationship of the bonded and nonbonded regions of the gathered regions of this layered composite with respect to the dimensions of both the unperturbed starting first layer and the at least one additional layer.

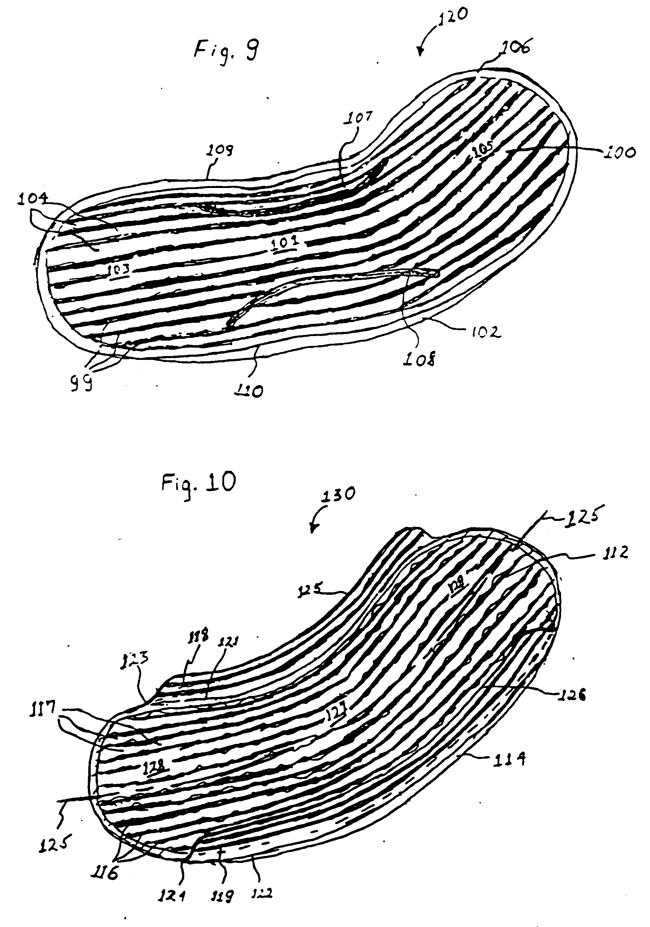

Figure 9 shows a perspective view of an absorbent product of this invention, here a sanitary napkin **120** covered by a perforated fluid permeable top layer **100**, a fluid impermeable bottom layer **102** and an absorbent core (not shown) therebetween, where at least top layer **100** is made of the gathered, layered composite material of this invention; top layer **100**, here exhibiting bonded **99** and nonbonded **104** areas (the at least one additional layer not being shown). Absorbent core **104** is comprised of an absorbent, for example woodpulp, and has more absorbent, thereby being thicker in approximately the central portion **101**, i.e., approximately the central third, then becoming thinner by tapering down toward the transverse ends **103, 105** of napkin **120**. Napkin **120** is distinguished by having top layer **100** sealed along its entire periphery **106** to bottom layer **102**, wherein bottom layer **102** may be comprised of a nonperforate material comprised of the layered composite material of this invention. Napkin **120** is further distinguished by having a pair of deeply embossed concavedly curving channels **107, 108,** toward and adjacent each of two lateral sides **109, 110,** respectively, along and outward of central portion **101** of napkin **120**, the deeply embossed channels **107, 108**, being a highly densified region containing top layer **100** and a significant depth of the absorbent comprising absorbent core **104**. The thickness of absorbent core **104** in central portion **101** between the pair of channels **107, 108** is at least equal to or greater than the thickness of central portion **101** between pair of channels **107, 108** and lateral sides **109, 110**. Napkin **120** also comprises a positioning adhesive (not shown), on the garment side of bottom layer **102**, for attaching napkin **120** to an undergarment, positioning adhesive being protected until use with a release paper (not shown). Absorbent core **104** may optionally contain as distinct layers, gradients, or as homogeneous dispersions, any or all of absorbents such as tissue, sphagnum moss and superabsorbent.

Figure 10 shows a perspective view of an absorbent product of this invention, here a sanitary napkin **130** covered by a fluid permeable top layer **112**, a fluid impermeable bottom layer **113** and an absorbent core (not shown) therebetween, where top layer **112** and/or bottom layer **114** may be made of the gathered, layered composite of this invention; top layer **112**, here exhibiting bonded **116** and nonbonded **117** areas (the at least one additional layer not being shown). Napkin **130** also comprises right and left longitudinally extending cuffs **118, 119** each of the cuffs being attached along their respective base portions **121, 122** to the right and left lateral sides **123, 124** of napkin, respectively, such that the distal ends **125, 126** of cuffs **118, 119** extend outward from right and left lateral sides **123, 124** of napkin **130**. Top layer **112** and bottom layer **114** are attached to each other in a flange seal **122** and to base portions **121, 122** of cuffs **118**, **119**. Cuffs **118, 119** may be optionally longitudinally attached along their respective base portions **121, 122** to napkin **130**: anywhere, between the respective lateral sides **123, 124** and the longitudinal centerline **125**; along and above or below top layer **112**, but not including longitudinal centerline **125**; along and above or below bottom layer **114**, including longitudinal centerline **125**; in such fashion that ensures that cuffs **118, 119** extend at least outward from lateral sides **123, 124**. Cuffs **118, 119** in this example may also be covered with a nonwoven or, as shown here, with the gathered, layered composite material of this invention; the gathered, layered composite material here exhibiting bonded **116** and nonbonded **117** areas (the at least one additional layer not being shown). Cuffs **118, 119** may additionally be made of or contain in or on the cuffs other materials such as films, nonwovens and foam, the nonwoven or foam being preferably of a highloft material. Cuffs **118, 119** may also additionally contain elastic materials in or on the cuffs. Absorbent core **116** in Figure 10 has a roughly rectangular outline, and is thicker in the middle **127** than lateral sides **123, 124** or than the transverse ends **128, 129**. Absorbent core **116** may have other outline shapes such as hourglass, dogbone or oval. Absorbent core **116**, proceeding from the absorbent facing side of top layer **112**, may further comprise a transfer layer of a low density nonwoven material, an auxiliary absorbent layer containing sphagnum moss and a main absorbent layer such as of airlaid pulp. Napkin **130** also comprises positioning adhesive (not shown), on the garment side of bottom layer **114**, for attaching napkin **130** to an undergarment, positioning adhesive being protected until use by a release paper (not shown). Absorbent core may optionally contain as distinct layers, gradients, or as homogeneous dispersions, any or all of tissue, sphagnum moss and superabsorbent. Embodiments of napkins with cuffs such as are described herein are described in Patent Applications, to Salerno et al, commonly assigned, entitled, "Absorbent Article Having Compliant Cuffs" and "Stabilized Absorbent Article", are herein incorporated in their entirety.

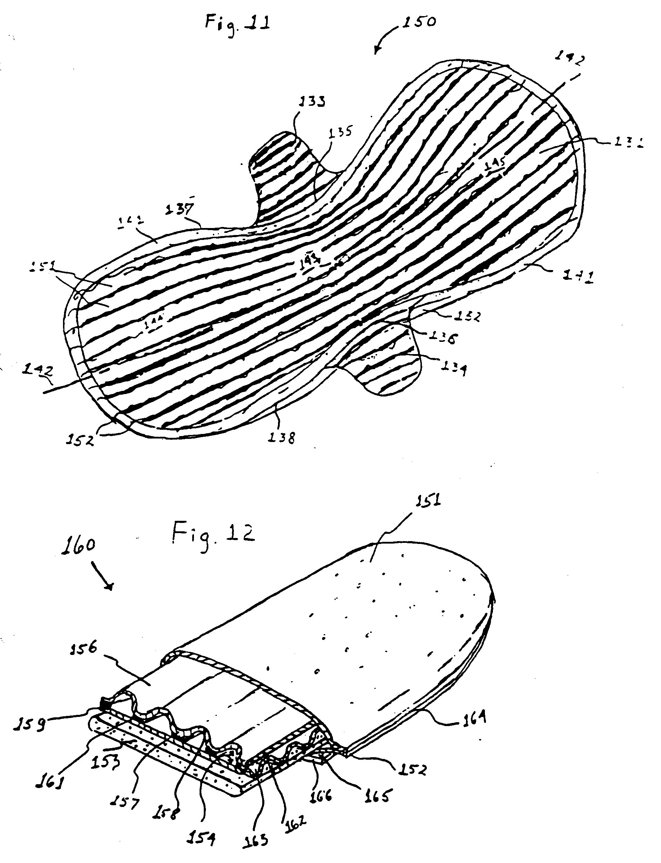

Figure 11 shows a perspective view of an absorbent product of this invention, a sanitary napkin **150** covered by a fluid permeable top layer **131**, a fluid impermeable bottom layer **132** and an absorbent core (not shown) therebetween. Napkin **150** also comprises right and left longitudinally extending wings or tabs **133, 134** each of the wings or tabs **133, 134** being attached along their respective base portions **135, 136** to the right and left lateral sides **137, 138** of napkin **150,** respectively, such that the distal ends **139, 140** of wings or tabs **133, 134**, extend outward from the right and left lateral sides **137, 138** of sanitary napkin **150**. Top layer **131** and bottom layer **132** are attached to each other in a flange seal **141** and to the base portions of wings or tabs **133, 134**. Wings or tabs **133, 134** in this example are also covered with the layered composite material of this invention. The wings or tabs can optionally be attached to the sanitary napkin: longitudinally along and above or below the cover between and including the center of the napkin and adjacent the lateral sides; longitudinally anywhere along the lateral sides of the napkin; and longitudinally along and above or below the barrier film backsheet. Wings or tabs **133**, **134** may be longitudinally attached, alternatively along their respective base portions **135**, **136** anywhere, between the respective lateral sides **137**, **138** and adjacent to but not including the longitudinal centerline **142**; such that wings or tabs **133**, **134** extend at least outward from lateral sides **137**, **138**. Absorbent core **133** in Figure 11 has an hourglass outline and is thicker in the middle region **143** than the lateral sides **137**, **138** or than the transverse ends **144**, **145**. Absorbent core **133** may further comprise, proceeding from the absorbent facing side of top layer **131**, a transfer layer made of a low density nonwoven material, an auxiliary absorbent layer comprising sphagnum moss and a main absorbent layer such as of airlaid pulp. Napkin **150** also comprises positioning adhesive (not shown), on the garment side of bottom layer **132**, for attaching napkin **150** and wings or tabs **133**, **134** to an undergarment, positioning adhesive being protected until use with a release paper (not shown). Absorbent core **133** may optionally contain as distinct layers, gradients, or as homogeneous dispersions, any or all of absorbents such as tissue, sphagnum moss and superabsorbent. Embodiments of napkins with wings such as are described herein are described in US Patent Application, Serial No. 198,809 entitled, "Body Conforming
Absorbent Article" to McCoy et al, and commonly assigned.

Figure 12 shows a partial cross section of a perspective view of an absorbent product of this invention, here a sanitary napkin **160**, covered by a perforated fluid permeable top layer **151**, a fluid impermeable bottom layer **152** and an absorbent core **153**, therebetween, absorbent core **153** further comprising and insert **154** made of the gathered, layered composite material of this invention. Top layer **151** and bottom layer **152** are attached to each other in a flange seal **164**. Napkin **160** also comprises positioning adhesive **165**, on the garment side of bottom layer **152**, for attaching napkin **160**, positioning adhesive **165** being protected until use with a release paper **166**. Insert **154** may be positioned anywhere within absorbent core **153** between top layer **151** and bottom layer **152**, and may substitute for any or all off the components of absorbent core **153**. Thus, when insert **154** is comprised of at least one perforate component layer, it has been found useful as an intervening fluid transfer layer to wick fluids both laterally and vertically and for storage or containment structures for ingredients such as deodorants, fragrances, medicaments and superabsorbents. Here insert **154** is positioned between top layer **151** and absorbent core **153**, and has at least one perforate component layer. Insert **154** is comprised of a first layer **156** and an at least one additional layer **159** attached to each other by spaced apart bonding means **161**; wherein first layer is comprised of bonded areas **157** and nonbonded areas **158**, and at least one additional layer **159** layer is comprised of bonded areas **162** and nonbonded areas **163**. Since insert **154** here may act as a fluid transfer layer, absorbent core **153** may comprise an auxiliary absorbent layer comprising such as sphagnum moss and a main absorbent layer such as of airlaid pulp; and may optionally contain as distinct layers, gradients, or as homogeneous dispersions, any or all of absorbents such as tissue, sphagnum moss and superabsorbent. When the layered composite material comprising the insert is comprised completely of perforate or nonperforate component layers or has both perforate and nonperforate component layers, it has also been found useful as deformation and crush resistant structures.

### EXAMPLES

In addition to the preceding descriptions, and as additionally illustrated by the figures, the gathered layered composite of the invention is further illustrated, in the following Table, "Manufacture of Laminated Composite Material", by examples of manufacturing runs used to make the gathered, layered composite materials, without meaning to be limited by such examples. The Table describes manufacturing runs wherein a Layer 1 was tensioned and concomitantly narrowed and, while in a tensioned and narrowed state, was adhered to a Layer 2 that had parallel spaced apart hotmelt adhesive lines in the machine direction. The examples of Layer 1 described in the Table include: a perforate high 3D profile film, made of polyethylene, as described in previously cited Canadian Patent Application 2,130,176 and in US Patent Application 08/522,722 and European Patent Applications 96.929077.4 and 96.931423.6; a perforate low 3D profile film as made by applying vacuum to a polyethylene film supported on a perforated cylindrical drum; a flat perforate film made from a coextruded film comprised of polyethylene and polyethylene polyvinyl acetate copolymer as described in previously cited US Patents 4,806,411 and 4,859,519; and various nonperforate polyethylene films of different basis weights.

Tensioning and concomitant narrowing of Layer 1 was accomplished by the difference in speeds between cylinders that transferred Layer 1, wherein Layer 1 was pulled and thereby stretched by the cylinder driven by Drive 2 which rotated at a faster speed than the cylinder driven by Drive 1; and wherein the cylinder driven by Drive 2 brought Layer 1 to the nip where it was laminated to Layer 2 and subsequently allowed to relax from its tensioned and narrowed state. In the set of columns, labelled "Width of Layer 1", the starting width of Layer 1 is compared first with the tensioned and thereby narrowed width of Layer 1 and then with the relaxed width of Layer 1, which is the same as its gathered width in the layered laminated composite. It should be noted that although the gathered width is smaller than the starting width, the actual width of material contained in gathered Layer 1 is essentially the same as that of the starting ungathered width, i.e., the recovery of Layer 1 is essentially 100%. In the set of columns, labelled "Length of Layer 1", the starting length of Layer 1 is compared first with the tensioned and thereby stretched length of Layer 1 and then with the relaxed length of Layer 1, which is the same as its gathered length in the gathered layered composite. It should be noted: that the gathered length of Layer 1 is quite similar to its starting length, since essentially all the gathers of Layer 1, wherein the lines of adhesive are parallel to the direction of tensioning, occur in the width, i.e., in the crossmachine direction; and that the recovery of Layer 1 in the length is essentially 100%.

Layer 2 in these manufacturing runs included: a single ply of polypropylene spunbond nonwoven (NW) of different basis weights or of tissue (Tiss) of different basis weights; double plies of nonwoven and tissue; and triple plies of a nonwoven ply and two plies of tissue.

The Table also gives, for Layer 2, the spacing between hotmelt adhesive lines as well as the number of gathers/in. contained in Layer 1 in the composite, the latter being determined predominantly by the degree of gathering imposed on Layer 2 by the stretched and relaxed Layer 1 and the number of gathers in Layer 1 being determined to a great degree by the spacing between the adhesive lines on Layer 2.

In describing the present invention, certain embodiments have been used for purposes of illustration; however, other embodiments or modifications within the scope of the invention will readily occur to those skilled in the art after reading this disclosure. The invention is accordingly not to be limited to the specific embodiments described and illustrated herein, but only in accordance with the appended claims.

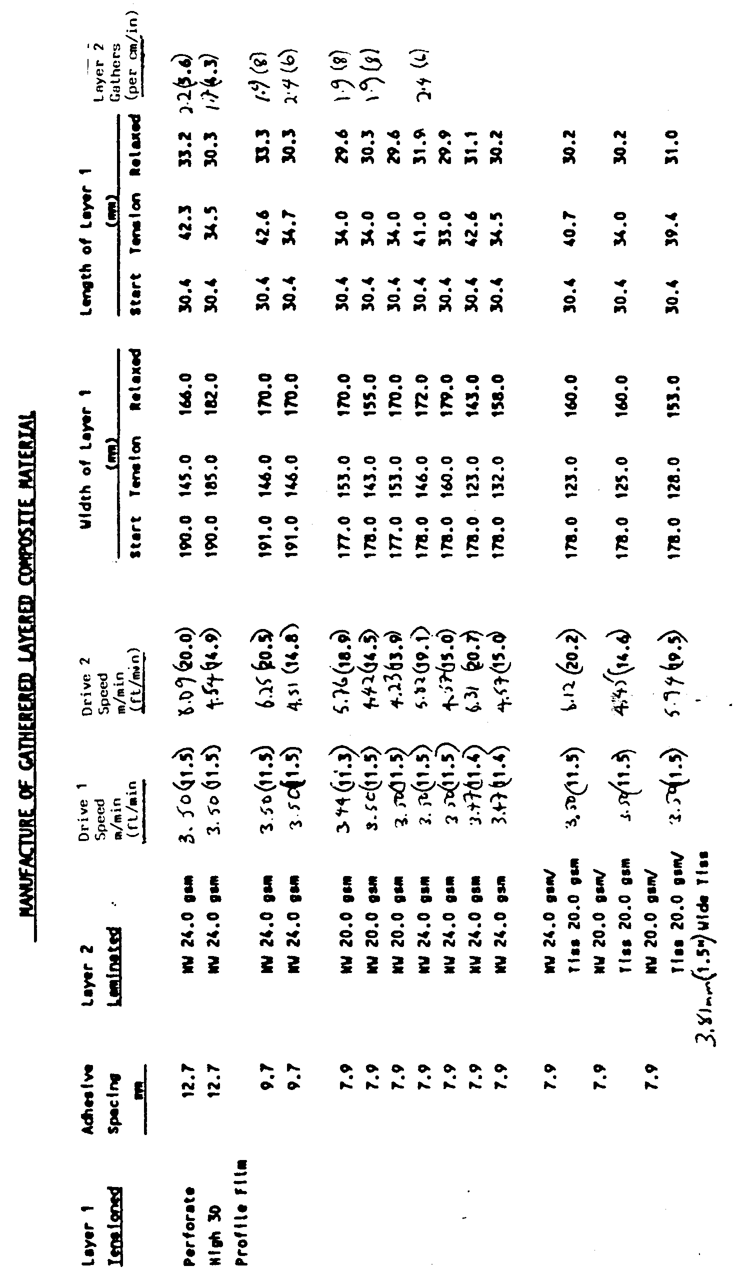

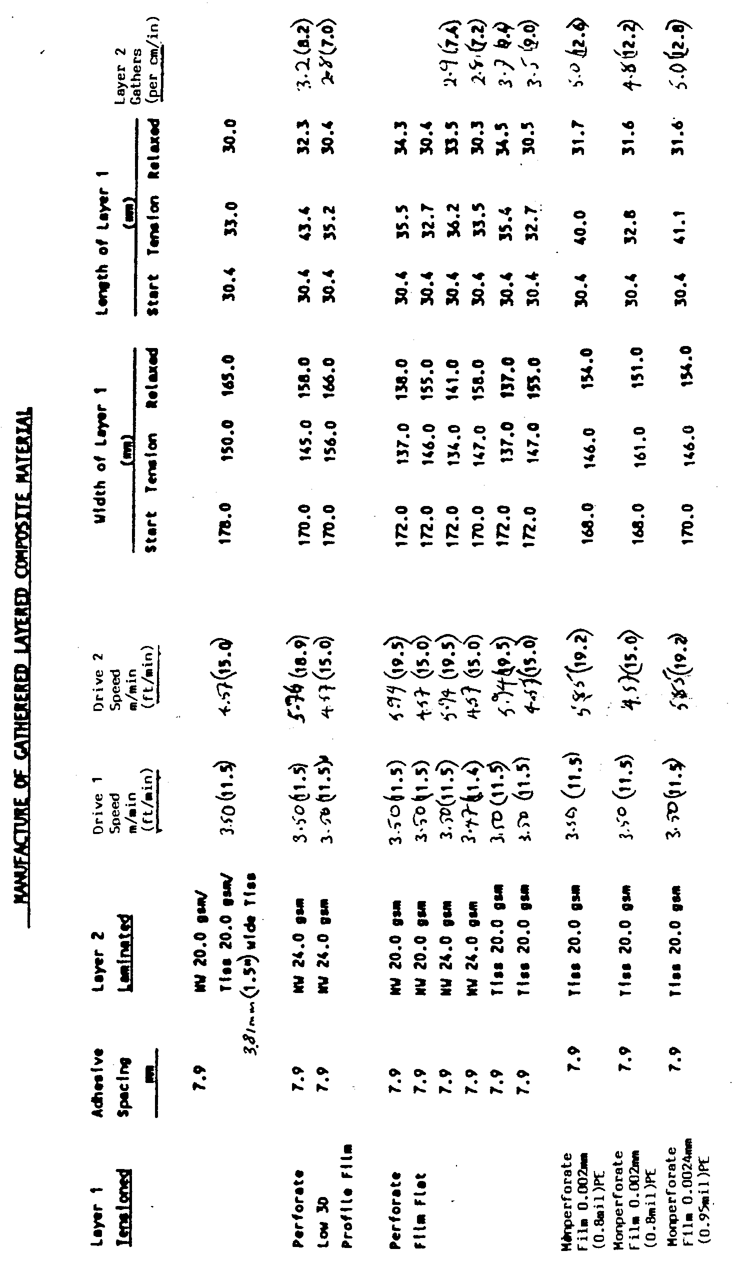

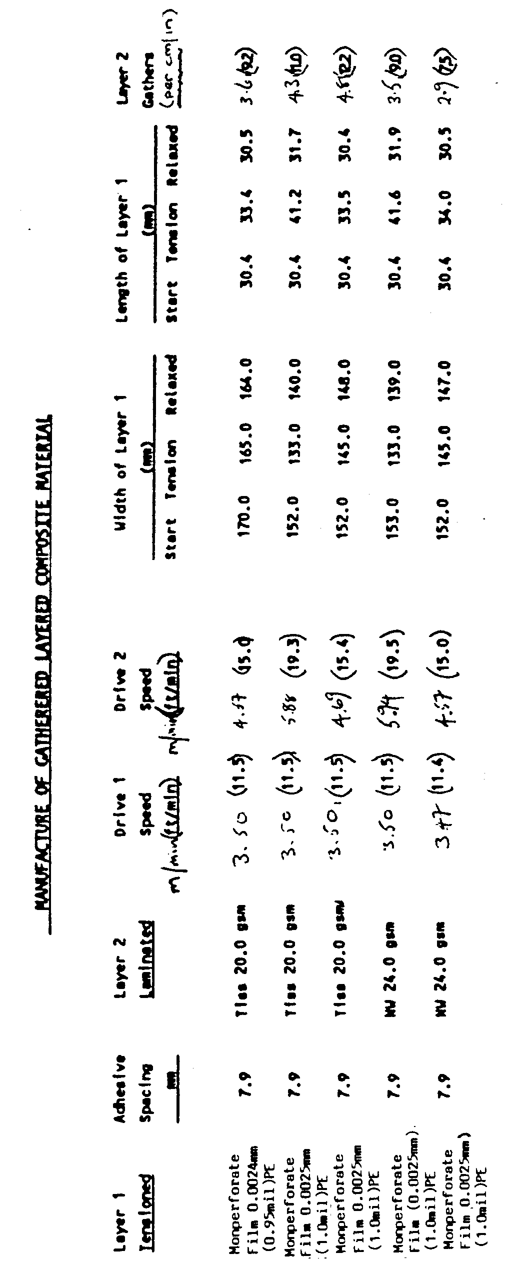

## Claims

1. A gathered, layered composite material comprising:
a first layer of a flexible, reversibly elongatable material, bonded at spaced apart positions along a substantial portion of the first layer, to at least one additional layer of a flexible nontensioned material;
wherein both the first layer and the at least one additional layer are gathered, the gathers of one layer being aparallel to the gathers of the other layer.

2. The gathered, layered composite material according to claim 1, wherein the first layer is a material selected from the group consisting of fabric, film and foam.

3. The gathered, layered composite material according to claim 2, wherein the film is selected from the group consisting of nonperforate films, perforate films, embossed films and nonembossed films.

4. The gathered, layered composite material according to claims 2, wherein the fabric, film or foam is nonelastic.

5. A method of forming a gathered, layered composite material comprising:
tensioning a first layer of a flexible, elongatable material in an amount for it to be reversibly elongated in the direction of tensioning and for it to be reversibly narrowed in a direction coplanarly orthogonal to the direction of tensioning;
bonding the first layer, while it is being concomitantly tensioned in the direction of tensioning and narrowed orthogonal to the direction of tensioning, by a bonding means at spaced apart positions along a substantial portion of the first layer, to at least one additional layer comprised of a flexible nontensioned material, to form a layered composite material;
and then releasing both the tensioning, and the narrowing of the first layer, to form a three dimensional, layered composite material, wherein both the first layer and the at least one additional layer are gathered, the gathers of one layer being aparallel to the gathers of the other layer.

6. The method according to claim 5, wherein the first layer is a material selected from the group consisting of fabric, film and foam.

7. The method according to claim 6, wherein the film is selected from the group consisting of nonperforate films, perforate films, embossed films and nonembossed films.

8. The method according to claim 6, wherein the fabric, film or foam is nonelastic.

9. The method according to claim 6, wherein the fabric or film is made of materials selected from the group consisting of polyethylene, polypropylene, noncrosslinked ethylene vinyl acetate copolymers, polyamides such as nylon 6 and nylon 6,6, polyesters, polyvinylidene chloride, polyvinyl chloride, polyester polyurethanes, polyether polyurethanes, and blends, coextruded laminates, layered laminates of such materials, and combinations thereof.

10. A sanitary napkin having a fluid pervious top layer, a fluid impervious bottom layer, and an absorbent core therebetween, wherein the top layer and/or the bottom layer is comprised of a gathered, layered composite material, the material being according to any one claims 1 to 4.

11. A sanitary napkin, having a longitudinal centerline, a pair of lateral sides and a pair of transverse ends, the napkin comprising a fluid pervious top layer, a fluid impervious bottom layer, and an absorbent core therebetween;
the napkin having within the central portion a pair of deeply embossed channels, the channels including the top layer and at least part of the absorbent core; each channel curving concavedly toward each pair of lateral sides, respectively;
the absorbent core being thicker in the central portion, and tapering to less thickness form the central portion to the transverse ends; and being further characterized by having the thickness of the central portion between the channels to be at least equal to the thickness of the central portion between the channels and the lateral sides; and
wherein the top layer and/or the bottom layer is comprised of a gathered, layered composite material, the material being according to any one of claims 1 to 4.

12. A sanitary napkin, having a longitudinal centerline, a pair of lateral sides and a pair of transverse ends, the napkin comprising a fluid pervious top layer, a fluid impervious bottom layer, and an absorbent core therebetween;
and a pair of cuffs, each having a base portion and a distal end, each cuff longitudinally extending and attached, along its respective base portion, to one or a combination of the top layer, the bottom layer and respective lateral side; the longitudinally extending attachment being anywhere, between the longitudinal centerline; such that the distal ends of the cuffs extend at least outward from the lateral sides of the napkin;
and wherein any or all of the top layer, the bottom layer and the cuffs is comprised of a gathered, layered composite material, the material being according to any one of claims 1 to 4.

13. A sanitary napkin, having a longitudinal centerline, a pair of lateral sides and a pair of transverse ends, the napkin comprising a fluid pervious top layer, a fluid impervious bottom layer, and an absorbent core therebetween; and
a pair of wings, each having a base portion and a distal end, each wing longitudinally extending and attached, along its respective base portion, to one or a combination of the top layer, the bottom layer and the respective lateral side; the longitudinally extending attachment being anywhere, between the lateral side and the longitudinal centerline; such that the distal ends of the wings extend at least outward form the lateral sides of the napkin; and
and wherein any or all of the top layer, the bottom layer and the wings is comprised of a gathered, layered composite material, the material being according to any one of claims 1 to 4.

14. A sanitary napkin having a fluid pervious top layer, a fluid impervious bottom layer, an absorbent core therebetween, and at least one insert; the insert being located between the top layer and the bottom layer; and wherein any or all of the top layer, the bottom layer and the insert is comprised of a gathered, layered composite material, the material being according to any one of claims 1 to 4.

## Patentansprüche

1. Gerafftes Schicht-Verbundmaterial, mit:
einer ersten Schicht aus einem flexiblen, reversibel dehnbaren Material, das an beabstandeten Positionen entlang einem wesentlichen Abschnitt der ersten Schicht mit wenigstens einer zusätzlichen Schicht eines flexiblen, nicht unter Spannung stehenden Materials verbunden ist,
wobei sowohl die erste Schicht als auch die wenigstens eine zusätzliche Schicht gerafft sind, wobei die Fältelungen einer Schicht nicht parallel zu den Fältelungen der anderen Schicht sind.

2. Gerafftes Schicht-Verbundmaterial nach Anspruch 1, bei dem die erste Schicht ein Material ist, das aus der Gruppe bestehend aus Textilien, Film und Schaum ausgewählt ist.

3. Gerafftes Schicht-Verbundmaterial nach Anspruch 2, bei dem der Film aus der Gruppe bestehend aus nicht perforierten Filmen, perforierten Filmen, geprägten Filmen und nicht geprägten Filmen ausgewählt ist.

4. Gerafftes Schicht-Verbundmaterial nach Anspruch 2, bei dem das Textil, der Film oder der Schaum unelastisch ist.

5. Verfahren zum Bilden eines gerafften, geschichteten Komposit-Materials, durch:
Spannen einer ersten Schicht aus einem flexiblen, dehnbaren Material um einen Betrag, daß sie reversibel in der Spannrichtung gedehnt wird und daß sie reversibel in einer Richtung koplanar orthogonal zu der Spannrichtung verengt wird;
Verbinden der ersten Schicht, während sie gleichzeitig in der Spannrichtung gespannt und orthogonal zu der Spannrichtung verengt wird, durch ein Bindemittel an beabstandeten Positionen entlang einem wesentlichen Abschnitt der ersten Schicht mit wenigstens einer zusätzlichen Schicht, die aus einem flexiblen ungespannten Material besteht, um ein Schicht-Verbundmaterial zu bilden;
und dann Lösen sowohl der Spannung als auch des Verengens der ersten Schicht, um ein dreidimensionales Schicht-Verbundmaterial zu bilden, bei dem sowohl die erste Schicht als auch die wenigstens eine zusätzliche Schicht gerafft sind, wobei die Fältelungen einer Schicht nicht parallel zu den Fältelungen der anderen Schicht sind.

6. Verfahren nach Anspruch 5, bei dem die erste Schicht ein Material ist, das aus der Gruppe bestehend aus Textilien, Film und Schaum ausgewählt ist.

7. Verfahren nach Anspruch 6, bei dem der Film aus der Gruppe bestehend aus nicht perforierten Filmen, perforierten Filmen, geprägten Filmen und nicht geprägten Filmen ausgewählt ist.

8. Verfahren nach Anspruch 6, bei dem das Textil, der Film oder der Schaum unelastisch ist.

9. Verfahren nach Anspruch 6, bei dem das Textilien oder der Film aus Materialien hergestellt ist, die aus der Gruppe bestehend aus Polyethylen, Polypropylen, unvernetzten Ethylenvinylacetat-Copolymeren, Polyamiden, so wie Nylon 6, Nylon 6,6, Polyester, Polyvinylidenchlorid, Polyvinylchlorid, Polyesterpolyurethanen, Polyetherpolyurethanen und Mischungen, koextrudierten Laminaten, Schicht-Laminaten solcher Materialien und Kombinationen daraus ausgewählt ist.

10. Damenbinde mit einer flüssigkeitsdurchlässigen oberen Schicht, einer flüssigkeitsundurchlässigen unteren Schicht und einem dazwischen liegenden absorbierenden Kern, bei der die obere Schicht und/oder die untere Schicht aus einem gerafften, geschichteten Komposit-Material besteht, wobei das Material aus einem nach einem der Ansprüche 1 bis 4 besteht.

11. Damenbinde mit einer Längsmittellinie, einem Paar Seiten und einem Paar quer verlaufender Enden, wobei die Binde eine flüssigkeitsdurchlässige obere Schicht, eine flüssigkeitsundurchlässige untere Schicht und einen dazwischen liegenden absorbierenden Kern aufweist;
wobei die Binde innerhalb des Mittelabschnittes ein Paar tiefgeprägter Kanäle aufweist, wobei die Kanäle die obere Schicht und wenigstens einen Teil des absorbierenden Kerns umfassen, wobei jeder Kanal sich jeweils konkav in Richtung auf jedes Paar Seiten krümmt;
wobei der absorbierende Kern im Mittelabschnitt dicker ist und sich vom Mittelabschnitt zu den quer verlaufenden Enden auf geringere Dicke abschrägt und weiter gekennzeichnet dadurch, daß die Dicke des Mittelabschnitts zwischen den Kanälen wenigstens gleich der Dicke des Mittelabschnitts zwischen den Kanälen und den Seiten ist; und
bei der die obere Schicht und/oder die untere Schicht aus einem gerafftem, geschichteten Komposit-Material besteht, wobei das Material eines nach einem der Ansprüche 1 bis 4 ist.

12. Damenbinde mit einer Längsmittellinie, einem Paar Seiten und einem Paar quer verlaufender Enden, wobei die Binde eine flüssigkeitsdurchlässige obere Schicht, eine flüssigkeitsundurchlässige untere Schicht und einen dazwischen liegenden absorbierenden Kern aufweist;
und mit einem Paar Manschetten, die jeweils einen Basisabschnitt und ein distales Ende haben, wobei sich jede Manschette in Längsrichtung erstreckt und entlang ihrem jeweiligen Basisabschnitt an der oberen Schicht, der unteren Schicht oder der jeweiligen Seite oder einer Kombination befestigt ist; wobei die sich in Längsrichtung erstreckende Befestigung irgendwo zwischen der Längsmittellinie ist; derart, daß die distalen Enden der Manschetten sich von den Seiten der Binde wenigstens nach außen erstrecken;
und bei der die obere Schicht, die untere Schicht oder die Manschetten oder alle diese aus einem gerafftem, geschichteten Komposit-Material bestehen, wobei das Material eines nach einem der Ansprüche 1 bis 4 ist.

13. Damenbinde mit einer Längsmittellinie, einem Paar Seiten und einem Paar quer verlaufender Enden, wobei die Binde eine flüssigkeitsdurchlässige obere Schicht, eine flüssigkeitsundurchlässige untere Schicht und einen dazwischen liegenden absorbierenden Kern aufweist; und
mit einem Paar Flügel, die jeweils einen Basisabschnitt und ein distales Ende haben, wobei sich jeder Flügel in Längsrichtung erstreckt und entlang seinem jeweiligen Basisabschnitt an der oberen Schicht, der unteren Schicht und der jeweiligen Seite oder einer Kombination dieser befestigt ist; wobei die sich in Längsrichtung erstreckkende Befestigung irgendwo zwischen der Seite und der Längsmittellinie ist; derart, daß die distalen Enden der Flügel sich von den Seiten der Binde wenigstens nach außen erstrecken; und
bei der die obere Schicht, die untere Schicht oder die Flügel oder alle diese aus einem gerafftem, geschichteten Komposit-Material bestehen, wobei das Material eines nach einem der Ansprüche 1 bis 4 ist.

14. Damenbinde mit einer flüssigkeitsdurchlässigen Deckschicht, einer flüssigkeitsundurchlässigen unteren Schicht, einem dazwischen liegenden absorbierenden Kern und wenigstens einer Einlage; wobei sich die Einlage zwischen der Deckschicht und der unteren Schicht befindet und wobei die obere Schicht, die untere Schicht oder die Einlage oder alle diese aus einem gerafftem geschichteten Komposit-Material besteht, wobei das Material eines nach einem der Ansprüche 1 bis 4 ist.

## Revendications

1. Matériau composite à couches, froncé qui comprend:
une première couche d'un matériau apte à s'allonger de manière réversible, souple, fixée en des positions espacées à intervalles le long d'une portion importante de la première couche, à au moins une couche supplémentaire d'un matériau non tendu, souple;
dans lequel l'ensemble de la première couche et de la couche supplémentaire au nombre d'au moins une est froncé , les fronces d'une couche étant aparallèles aux fronces de l'autre couche.

2. Matériau composite à couches, froncé selon la revendication 1, dans lequel la première couche est un matériau choisi dans le groupe comprenant les tissus, films et mousses.

3. Matériau composite à couches, froncé selon la revendication 2, dans lequel le film est choisi dans le groupe comprenant les films non perforés, les films perforés, les films gaufrés et les films non gaufrés.

4. Matériau composite à couches, froncé selon la revendication 2, dans lequel le tissu, film ou mousse est non élastique.

5. Procédé de formation d'un matériau composite à couches, froncé qui consiste à:
tendre une première couche d'un matériau apte à s'allonger, souple en une quantité telle qu'il soit allongé de manière réversible dans la direction de tension et qu'il soit rétréci de manière réversible dans une direction coplanairement orthogonale à la direction de tension;
fixer la première couche, alors qu'elle est simultanément tendue dans la direction de tension et rétrécie orthogonalement à la direction de tension, par un moyen de fixation en des positions espacées à intervalles le long d'une partie importante de la première couche, à au moins une couche supplémentaire constituée d'un matériau souple non tendu, pour former un matériau composite à couches;
et ensuite relacher à la fois la tension et le rétrécissement de la première couche, pour former un matériau composite à couches, tridimensionnel dans lequel l'ensemble de la première couche et de la couche supplémentaire au nombre d'au moins une est froncé, les fronces d'une couche étant aparallèles aux fronces de l'autre couche.

6. Procédé selon la revendication 5, dans lequel la première couche est un matériau choisi dans le groupe comprenant les tissus, films et mousses.

7. Procédé selon la revendication 6, dans lequel le film est choisi dans le groupe comprenant les films non perforés, les films perforés, les films gaufrés et les films non gaufrés.

8. Procédé selon la revendication 6, dans lequel le tissu, le film ou la mousse est non élastique.

9. Procédé selon la revendication 6, dans lequel le tissu ou le film est fait de matériaux choisis dans le groupe comprenant le polyéthylène, le polypropylène, les copolymères éthylène - acétate de vinyle non réticulés, les polyamides tels que le Nylon 6 ou le Nylon 6,6, les polyesters, le poly(chlorure de vinylidène), le poly(chlorure de vinyle), les polyesters polyuréthannes , les polyéthers polyuréthannes et les mélanges, les stratifiés coextrudés, les stratifiés à couches de ces matériaux et leurs combinaisons.

10. Serviette hygiénique ayant une couche supérieure perméable aux fluides, une couche inférieure imperméable aux fluides et un intérieur absorbant entre elles, dans laquelle la couche supérieure et/ou la couche inférieure sont constituées d'un matériau composite à couches, froncé, le matériau étant conforme à l'une quelconque des revendications 1 à 4.

11. Serviette hygiénique ayant une ligne médiane longitudinale , une paire de bords latéraux et une paire d'extrémités transversales, la serviette comprenant une couche supérieure perméable aux fluides, une couche inférieure imperméable aux fluides et un intérieur absorbant entre elles;
la serviette ayant dans la portion centrale une paire de canaux profondément gaufrés, les canaux englobant la couche supérieure et au moins une partie de l'intérieur absorbant; chaque canal se courbant de manière concave vers chaque paire des bords latéraux respectivement;
l'intérieur absorbant étant plus épais dans la portion centrale , et s'effilant en une épaisseur moindre de la portion centrale vers les extrémités transversales; et encore caractérisée en ce que l'épaisseur de la portion centrale entre les canaux est au moins égale à l'épaisseur de la portion centrale entre les canaux et les bords latéraux; et
dans laquelle la couche supérieure et/ou la couche inférieure sont constituées d'un matériau composite à couches, froncé, le matériau étant conforme à l'une quelconque des revendications 1 à 4.

12. Serviette hygiénique ayant une ligne médiane longitudinale , une paire de bords latéraux et une paire d'extrémités transversales, la serviette comprenant une couche supérieure perméable aux fluides, une couche inférieure imperméable aux fluides et un intérieur absorbant entre elles;
et une paire de parements , chacun ayant une portion de base et une extrémité distale , chaque parement se prolongeant longitudinalement et étant fixé, le long de sa portion de base respective, à l'un d'entre,ou une combinaison de, la couche supérieure, la couche inférieure et le bord latéral respectif, la fixation se prolongeant longitudinalement étant n'importe où, entre la ligne médiane longitudinale; de telle sorte que les extrémités distales des parements se prolongent au moins vers l'extérieur à partir des bords latéraux de la serviette;
et dans laquelle n'importe lesquels ou la totalité d'entre la couche supérieure, la couche inférieure et les parements sont constitués d'un matériau composite à couches, froncé, le matériau étant conforme à l'une quelconque des revendications 1 à 4.

13. Serviette hygiénique ayant une ligne médiane longitudinale , une paire de bords latéraux et une paire d'extrémités transversales, la serviette comprenant une couche supérieure perméable aux fluides, une couche inférieure imperméable aux fluides et un intérieur absorbant entre elles; et
une paire de languettes , chacune ayant une portion de base et une extrémité distale , chaque languette se prolongeant longitudinalement et étant fixée, le long de sa portion de base respective, à l'un d'entre, ou une combinaison de la couche supérieure, la couche inférieure et le bord latéral respectif, la fixation se prolongeant longitudinalement étant n'importe où, entre le bord latéral et la ligne médiane longitudinale; de telle sorte que les extrémités distales des languettes se prolongent au moins vers l'extérieur à partir des bords latéraux de la serviette; et
dans laquelle n'importe lesquels ou la totalité d'entre la couche supérieure, la couche inférieure et les languettes sont constitués d'un matériau composite à couches, froncé, le matériau étant conforme à l'une quelconque des revendications 1 à 4.

14. Serviette hygiénique ayant une couche supérieure perméable aux fluides, une couche inférieure imperméable aux fluides , un intérieur absorbant entre elles et au moins un élément d'insertion; l'élément d'insertion étant situé entre la couche supérieure et la couche inférieure; et dans laquelle l'un quelconque ou l'ensemble de la couche supérieure , de la couche inférieure et de l'élément d'insertion est constitué d'un matériau composite à couches, froncé, le matériau étant conforme à l'une quelconque des revendications 1 à 4.
